# EUROPEAN PATENT APPLICATION

(11) **EP 1 690 949 A1**
(43) Date of publication of application: **16.08.2006**
(21) Application number: 06011392.5
(22) Date of filing: 16.09.2003
(51) Int. Cl.: C12Q 1/68, C12N 15/12, C07K 14/47, G01N 33/68

(54) **Variant of HNF-1alpha gene having a single nucleotide polymorphism and variant of protein encoded by the same**

(30) Priority: 16.09.2002 KR 20020056229
(62) Divisional of application: 03020967.0
(71) Applicant: SAMSUNG ELECTRONICS CO., LTD., Suwon-City, Kyungki-do (KR)
(72) Inventor: Lee, Yeon-su, 1307-101 Seongjeo Maeul, Ilsan-gu, Goyang-si, Kyungki-do (KR); Jeong, Sung-young, Samsung Advanced Institute, Kiheung-eub, Yongin-city, Kyungki-do (KR); Ahn, Kyu-jeung, Gangnam-gu, Seoul (KR); Kim, Byung-joon, Nowon-gu, Seoul (KR); Kim, Sook-young, Dong-gu, Daejeon-si (KR); Kim, Mi-kyung, Yuseong-gu, Daejeon (KR)
(74) Representative: Grünecker, Kinkeldey, Stockmair & Schwanhäusser Anwaltssozietät

(57) **Abstract**

A polynucleotide or nucleic acid fragment comprising a new single nucleotide polymorphism of a human HNF-1α gene. The polynucleotide or nucleic acid fragment includes a polymorphic site having adenine (A) at position 1699 of SEQ ID NO: 1 or having thymine (T) at position 29 of SEQ ID NO: 3, and more than 10 contiguous nucleotides set forth in SEQ ID NO: 1 or 3.

## Description

### BACKGROUND OF THE INVENTION

### 1. Field of the Invention

The present invention relates to a nucleic acid fragment comprising a polymorphic site of SEQ ID NO: 1 having adenine (A) nucleotide at position 1699, or a polymorphic site of SEQ ID NO: 3 having thymine (T) nucleotide at position 29, and comprising more than 10 contiguous nucleotides set forth in SEQ ID NO: 1 or 3, or a complement thereof, and a protein variant comprising the polymorphic site.

### 2. Description of the Related Art

Conventionally, various methods for analyzing a genetic variation have been widely used in the medical field to diagnose the occurrence of and/or propensity to a disease. The methods include a method comprising amplifying a gene by using an nucleic acid amplification method such as polymerase chain reaction (PCR), and analyzing the nucleotide sequence of the amplified gene by using nucleotide sequencing, hybridization or single strand conformational polymorphism (SSCP) analysis. For example, PCR and nucleotide sequencing are commercially used for BRCA gene probe. BRCA genes are known to be associated with breast cancer.

MODY3 genes give rise to maturity onset diabetes of the young, representing 10 - 30% or higher of type II diabetes (Yamada et al., *Diabetes* 48:645, 1997; Yoshiuchi et al., Human mutation 18:345, 2001; USP 6,187,533; WO9811254). ,

To date, a few cases of MODY3 gene mutation have been reported in Korea (Kim et al., Korean Diabetes Association, 23:793, 1999), and the reported cases are not linked with new mutations.

Therefore, there is demand for studies of novel mutations for disease diagnosis, investigation of disease mechanisms and drug discovery. In particular, there is an increasing need to screen MODY3 gene mutations specific to racial and geographical characteristics.

### SUMMARY OF THE INVENTION

The present invention provides a nucleic acid fragment comprising more than 10 contiguous nucleotides having a new mutation site, and complements thereof.

The present invention also provides allele-specific oligonucleotides hybridizing to all or some of nucleic acids comprising the new mutation site.

The present invention provides a method of analyzing a nucleic acid, comprising determining a nucleotide sequence of the nucleic acids comprising the new mutation site.

Also, the present invention provides a variant or fragment of a human HNF-1α polypeptide having an amino acid sequence consisting of 10 or more amino acids having mutations of the amino acids corresponding to the nucleic acids comprising the new mutation site.

The present invention provides a method for analyzing a protein comprising determining the mutations of the amino acids corresponding to the nucleic acid sequence comprising the new mutation site.

### BRIEF DESCRIPTION OF THE DRAWINGS

FIG. 1 shows a genomic DNA having a portion containing a mutant nucleotide of HNF-1α according to the present invention; and
FIG. 2 shows a genomic DNA having intron 8 containing a mutant nucleotide of HNF-1α according to the present invention.

### DETAILED DESCRIPTION OF THE INVENTION

A nucleic acid fragment according to the present invention comprises a polymorphic site of SEQ ID NO: 1 having adenine (A) at position 1699, or a polymorphic site of SEQ ID NO: 3 having thymine (T) at position 29, and comprises more than 10 contiguous nucleotides set forth in SEQ ID NO: 1 or 3 or complements thereof.

In detail, the nucleic acid fragment according to the present invention may include any polynucleotide that contains a polymorphic site of SEQ ID NO: 1 having a nucleotide A at position 1699 and more than 10 contiguous nucleotides set forth in SEQ ID NO: 1. The nucleic acid fragment according to the present invention may also include a HNF-1α gene itself.

The nucleic acid fragment preferably includes 10 to 100 contiguous nucleotides, more preferably 10 to 50 contiguous nucleotides, and most preferably 10 to 20 contiguous nucleotides, set forth in SEQ ID NO: 1 or complements thereof: The polymorphic site may be positioned at any portion of the fragment.

Also, the nucleic acid fragment according to the present invention may include any polynucleotide that contains more than 10 contiguous nucleotides set forth in SEQ ID NO: 3, which corresponds to a sequence of intron 8 positioned between exon 8 and exon 9 of human HNF-1α.

The nucleic acid may be DNA, RNA or PNA, and may be in the form of either single- or double-stranded nucleic acid. The nucleic acid fragment may be either a natural one or a synthesized one.

In the present invention, the term "polymorphism" refers to the occurrence of two or more alternative genomic sequences or alleles in a genetically determined group. A "polymorphic marker or site" is the locus at which the variation occurs. Preferably, a marker has at least two alleles having the frequency of greater than 1%, more preferably 10% to 20%, in a selected group. The polymorphic site may be a single base pair.

Also, the present invention provides an allele-specific oligonucleotide comprising a polymorphic site of SEQ ID NO: 1 having an A nucleotide at position 1699 or a polymorphic site of SEQ ID 3 having a T nucleotide at position 29, and hybridizing to the nucleotide of SEQ ID NO: 3, or complements thereof.

In the present invention, the term "allele-specific" refers to specifically hybridizing to each allele. For example, hybridizing is performed so as to specifically identify SNP of SEQ ID NO: 1 having an A nucleotide at position 1699. The hybridization is performed under stringent conditions, for example, conditions of 1 M or less in salt concentration and 25°C or higher in temperature. For example, conditions of 5xSSPE (750mM NaCl, 50mM Na Phosphate, 5mM EDTA, pH 7.4)and a temperature of 25~30°C are suitable for allele-specific probe hybridization.

In the present invention, the allele-specific oligonucleotide may be a probe. The term "probe" used herein means a hybridization probe, that is, oligonucleotide capable of sequence-specifically binding with a complementary strand of a nucleic acid. Such a probe includes peptide nucleic acids as described in Science 254, 1497-1500 (1991) by Nielsen et al. The probe according to the present invention, which is an allele-specific probe, is hybridized to DNA fragments derived from one component but is not hybridized to DNA fragments derived from different components since there is a polymorphic site in nucleic acid fragments derived from two components of the same species. In this case, hybridization conditions are significantly different in hybridization stringency between alleles. Thus, the hybridization conditions should be stringent enough to allow hybridization to only one allele. The probe according to the present invention is preferably disposed such that the central site, that is, position 7 in the case of a 15 nucleotide probe, or position 8 or 9 in the case of a 16 nucleotide probe, is aligned with a polymorphic site of the sequence, causing a significant difference in hybridization between alleles. The probe according to the present invention can be used in diagnostic methods for detecting alleles. The diagnostic methods include detection methods based on a nucleic acid hybridization, e.g., southern blot and in case where DNA chips are used to detect a particular nucleic acid, the probe may be provided as an immobilized form on a substrate of a DNA chip.

In the present invention, the allele-specific oligonucleotide may be a primer. The term "primer" used herein refers to a single stranded oligonucleotide capable of acting as a starting point of template-directed DNA synthesis under appropriate conditions (for example, in a buffered conditions containing four different nucleotide triphosphates and polymerases such as DNA or RNA polymerase or reverse transcriptase) and at an appropriate temperature. The appropriate length of the primer may vary according to the purpose of use, generally 15 to 30 nucleotides. In general, a shorter primer molecule requires a lower temperature to form a stable hybrid with a template. A primer sequence is not necessarily completely complementary with a template but should be complementary enough to hybridize to a template. The primer is preferably arranged such that the 3' end thereof is arranged with a polymorphic site of SEQ ID NO: 1 having a nucleotide A at position 1699 or a polymorphic site of SEQ ID NO: 3 having a nucleotide T at position 29. The primer hybridizes to a target DNA containing a polymorphic site, and start an allelic amplification in which the primer exhibits complete homology. The primer is used in pair with a second primer hybridizes at the opposite site. After amplification reaction, the amplified product is obtained from two primers, which means that there is a specific allelic form.

In accordance with another aspect of the present invention, there is provided a method of analyzing a nucleic acid comprising determining a nucleotide sequence at the polymorphic site of position 1699 of SEQ ID NO: 1 or of position 29 of SEQ ID NO: 3. The method may include isolating a nucleic acid such as gemonic DNA or RNA from a sample, and determining the sequence of the isolated nucleic acid and determining the nucleotide sequence at the polymorphic site of position 1699 of SEQ ID NO: 1 or of position 29 of SEQ ID NO: 3. If the analysis result shows that the nucleotide sequence at the polymorphic site of position 1699 of SEQ ID NO: 1 is a nucleotide other than guanine (G) or the nucleotide sequence at the polymorphic site of position 29 of SEQ ID NO: 3 is a nucleotide other than cytosine (C), it is determined that there is an increased risk of maturity onset diabetes of the young (MODY). Here, the nucleotide sequence is determined by general sequencing methods, for example, a dideoxy method (Sambrook et al.; Molecular Cloning, A Laboratory Manual, 2^{nd} Ed. CSHP, New York 1989), or using automated apparatuses.

A variant or fragment of human HNF-1α polypeptide according to the present invention, comprises a polymorphic site at which an amino acid at position 567 of SEQ ID NO: 2 is an amino acid other than valine (Val), preferably, isoleucine (IIe), and comprises more than 10 contiguous amino acids derived from the amino acid sequence of SEQ ID NO: 2. As set forth in SEQ ID NO: 2, the variant or fragment of human HNF-1α polypeptide according to the present invention, is preferably derived from the amino acid sequence of SEQ ID NO: 2 having Ile, instead of Val, at position 567.

The protein analyzing method of the present invention comprises determining the amino acid sequence at position 567 of SEQ ID NO: 2. The amino acid sequence is determined by general methods, for example, N- or C-terminal identification or using an apparatus such as MS/MS, or MALDI-TOF.

The present invention will now be described in more detail by referring to the following examples. However, these examples are provided for illustration only and the present invention is not limited to those examples.

### Examples

Genomic DNA was isolated from blood samples of 97 people who are clinically presumed as MODY patients, 10 exon and intron portions of MODY3 genes were amplified for base sequencing and MODY3 gene variations were detected.

### Example 1 : Amplification of 10 Exon DNA in human HNF-1α gene

Blood was collected from 97 patients, and genomic DNA was isolated therefrom using a QIAGEN plasmid midi kit (QIAGEN; Germany), 10 MODY3 exons were amplified using a PCR reaction solution (Table 1). Conditions of PCR amplification were 5 minutes (95°C) for initial denaturation, 30 cycles of 30 seconds (95°C) for denaturation, 15 seconds (62°C) for annealing and 30 seconds (72°C) for extension, and 3 minutes(72°C) for final extension. Primers used in PCR amplification are listed in Table 2.

Among primers listed in Table 2, T7 promoter sequence (SEQ ID NO: 24) was added into forward primers, and T3 promoter sequence (SEQ ID NO: 25) was added into 5' terminal of reverse primers.

**Table 1. Composition of PCR reaction solution**

| Ingredient | Volume(µℓ) |
|---|---|
| DNase, RNase-free water | 12.8 |
| dNTP mix (2.5 mM/nucleotide) | 2 |
| 10x Taq polymerase buffer | 2 |
| Primer set (10 pmol/primer) | 2 |
| Gemonic DNA (100∼ 1.0µg) | 1 |
| Taq polymerase (5 units/µℓ) | 0.2 |

**Table 2. PCR primers used in amplification of MODY3 gene**

| | Sequence |
|---|---|
| Mody 3 promoter sense(T7) | SEQ ID NO: 4 |
| Mody 3 promoter antisense(T3) | SEQ ID NO: 5 |
| Mody 3 exon1 sense(T7) | SEQ ID NO: 6 |
| Mody 3 exon1 antisense(T3) | SEQ ID NO: 7 |
| Mody 3 exon2 sense(T7) | SEQ ID NO: 8 |
| Mody 3 exon2 antisense(T3) | SEQ ID NO: 9 |
| Mody 3 exon3 sense(T7) | SEQ ID NO: 10 |
| Mody 3 exon3 antisense(T3) | SEQ ID NO: 11 |
| Mody 3 exon4 sense(T7) | SEQ ID NO: 12 |
| Mody 3 exon4 antisense(T3) | SEQ ID NO: 13 |
| Mody 3 exon5 sense(T7) | SEQ ID NO: 14 |
| Mody 3 exon5 antisense(T3) | SEQ ID NO: 15 |
| Mody 3 exon6 sense(T7) | SEQ ID NO: 16 |
| Mody 3 exon6 antisense(T3) | SEQ ID NO: 17 |
| Mody 3 exon7 sense(T7) | SEQ ID NO: 18 |
| Mody 3 exon7 antisense(T3) | SEQ ID NO: 19 |
| Mody 3 exon8 & 9 sense(T7) | SEQ ID NO: 20 |
| Mody 3 exon8 & 9 antisense(T3) | SEQ ID NO: 21 |
| Mody 3 exon10 sense(T7) | SEQ ID NO: 22 |
| Mody 3 exon10 antisense(T3) | SEQ ID NO: 23 |

Amplified products were purified using a QIAquick kit. The purified products were used as templates for nucleotide sequence analysis.

### Example 2 : Nucleotide sequence analysis of MODY3 gene

Nucleotide sequencing PCR was carried out using each purified primer for 10 MODY3 Exons by an ABI PRISM BigDye terminator cycle sequencing ready reaction kit (Applied Biosystem, U.S.A.) method, and the products were subjected to alcohol precipitation, followed by suspending in formamide, boiling at 5 minutes at 95°C,

immediately dipping into 4°C ice. Finally, sequence analysis was performed using an ABI PRISM 3700 Genetic Analyzer (Applied Blosystem, U.S.A.).

### Example 3 : Analysis for detecting mutations of MODY3 genes

DNA sequences resulting from the sequence analysis were compared with nucleotide sequences in NCBI database, and genetic mutations were analyzed using a DNAstar program (DNASTAR, Inc, U.S.A.).

10 exon sequences of MODY3 genes, which were amplified from a DNA sample of 97 patients diagnosed with type II diabetes, were used for analysis.

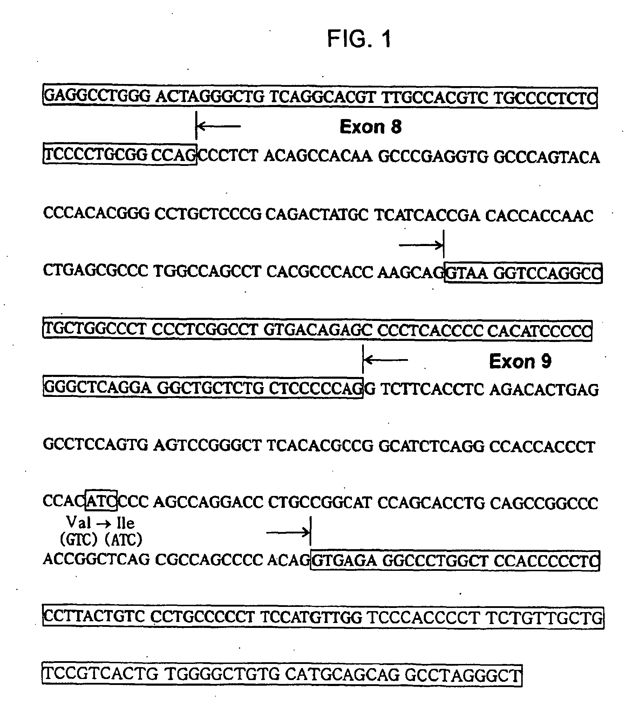

The result showed that mutations were found in 4 patients and a new mutation was found in one of the 4 patients. In other words, it was found that G at position 1699 of exon 9 in MODY3 gene of the patient was altered to A (FIG. 1). Also, it could be suggested that valine (Val) at position 567 in the amino acid sequence encoded by MODY3 gene was altered to isoleucine (Ile) (SEQ ID NO: 2).

### Example 4 : Nucleotide sequence analysis of Intron in MODY3 gene

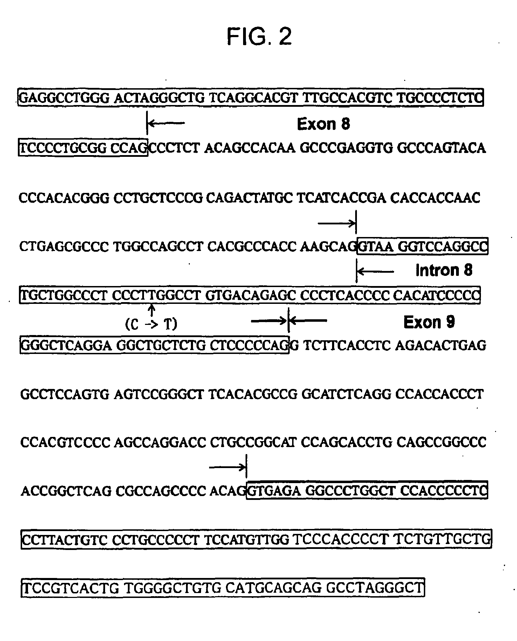

The same methods shown in Examples 1 through 3 were used for nucleotide sequence analysis of introns in HNF-1α. The analysis result showed that a new mutation was found in one patient. That is, C at position 29 of intron 8 positioned between exon 8 and exon 9 of HNF-1α was altered to T (FIG. 2).

Maturity onset diabetes of the young (MODY) cases among diabetic patients are relatively lower in Korea than in Europe in which MODY accounts for 10% of all diabetes cases, which is presumably because distributions of MODY genes are different depending on racial and geographical characteristics. Thus, further studies on MODY distributions must be conducted.

The nucleic acid fragment according to the present invention can be effectively used as a probe or primer in diagnosis of MODY.

The allele-specific oligonucleotide according to the present invention can be used as a probe or primer in diagnosis of MODY.

The variant or fragment human HNF-1α polypeptide according to the present invention can be effectively used as a probe or primer in diagnosis of MODY.

Also, the protein analyzing method according to the present invention can be effectively used as a probe or primer in diagnosis of MODY through peptide sequence analysis.

While the present invention has been particularly shown and described with reference to exemplary embodiments thereof, it will be understood by those of ordinary skill in the art that various changes in form and details may be made therein without departing from the spirit and scope of the present invention as defined by the following claims.

## Claims

1. A nucleic acid fragment comprising a polymorphic site of SEQ ID NO: 3 having thymine (T) at position 29, and comprising more than 10 contiguous nucleotides derived from nucleotide sequence set forth in SEQ ID NO: 3, or a complement thereof.

2. The nucleic acid fragment of claim 1, wherein the nucleic acid fragment comprises 10 to 100 contiguous nucleotides, or a complement thereof.

3. An allele-specific oligonucleotide hybridizing to the nucleic acid fragment of claim 1 or a complement thereof.

4. The allele-specific oligonucleotide of claim 3, wherein the oligonucleotide is a probe.

5. The allele-specific oligonucleotide of claim 3, wherein the oligonucleotide is a primer.

6. The allele-specific oligonucleotide of claim 5, wherein the 3'end of the primer is arranged with the polymorphic site of the nucleic acid fragment.

7. A method for analysing a nucleic acid comprising determining a nucleotide sequence of the polymorphic site at position 29 of SEQ ID NO: 3.

8. The method of claim 7, wherein if the nucleotide sequence of the polymorphic site at position 29 is T, it is determined that there is an increased risk for maturity onset of diabetes of the young (MODY).


SEQUENCE LISTING

<110>    Samsung Electronics Co. Ltd

<120>    A variant of HNF-1a gene having novel single nucleotide
         polymorphism and a variant protein encoded by the same

<160>    25

<170>    KopatentIn 1.71

<210>    1
<211>    1896
<212>    DNA
<213>    Homo sapiens

<220>
<221>    CDS
<222>    (1)..(1893)
<223>    amino acid sequence of HNF-1a

<400>    1
atg gtt tct aaa ctg agc cag ctg cag acg gag ctc ctg gcg gcc ctg          48
Met Val Ser Lys Leu Ser Gln Leu Gln Thr Glu Leu Leu Ala Ala Leu
  1               5                  10                  15

ctc gag tca ggg ctg agc aaa gag gca ctg atc cag gca ctg ggt gag          96
Leu Glu Ser Gly Leu Ser Lys Glu Ala Leu Ile Gln Ala Leu Gly Glu
             20                  25                  30

ccg ggg ccc tac ctc ctg gct gga gaa ggc ccc ctg gac aag ggg gag         144
Pro Gly Pro Tyr Leu Leu Ala Gly Glu Gly Pro Leu Asp Lys Gly Glu
         35                  40                  45

tcc tgc ggc ggc ggt cga ggg gag ctg gct gag ctg ccc aat ggg ctg         192
Ser Cys Gly Gly Gly Arg Gly Glu Leu Ala Glu Leu Pro Asn Gly Leu
     50                  55                  60

ggg gag act cgg ggc tcc gag gac gag acg gac gac gat ggg gaa gac         240
Gly Glu Thr Arg Gly Ser Glu Asp Glu Thr Asp Asp Asp Gly Glu Asp
 65                  70                  75                  80

ttc acg cca ccc atc ctc aaa gag ctg gag aac ctc agc cct gag gag         288
Phe Thr Pro Pro Ile Leu Lys Glu Leu Glu Asn Leu Ser Pro Glu Glu
                 85                  90                  95

gcg gcc cac cag aaa gcc gtg gtg gag acc ctt ctg cag gag gac ccg         336
Ala Ala His Gln Lys Ala Val Val Glu Thr Leu Leu Gln Glu Asp Pro
            100                 105                 110

tgg cgt gtg gcg aag atg gtc aag tcc tac ctg cag cag cac aac atc         384
Trp Arg Val Ala Lys Met Val Lys Ser Tyr Leu Gln Gln His Asn Ile
        115                 120                 125

cca cag cgg gag gtg gtc gat acc act ggc ctc aac cag tcc cac ctg         432
Pro Gln Arg Glu Val Val Asp Thr Thr Gly Leu Asn Gln Ser His Leu
    130                 135                 140

tcc caa cac ctc aac aag ggc act ccc atg aag acg cag aag cgg gcc         480
Ser Gln His Leu Asn Lys Gly Thr Pro Met Lys Thr Gln Lys Arg Ala
145                 150                 155                 160

gcc ctg tac acc tgg tac gtc cgc aag cag cga gag gtg gcg cag cag 528
Ala Leu Tyr Thr Trp Tyr Val Arg Lys Gln Arg Glu Val Ala Gln Gln
                165                 170                 175

ttc acc cat gca ggg cag gga ggg ctg att gaa gag ccc aca ggt gat 576
Phe Thr His Ala Gly Gln Gly Gly Leu Ile Glu Glu Pro Thr Gly Asp
            180                 185                 190

gag cta cca acc aag aag ggg cgg agg aac cgt ttc aag tgg ggc cca 624
Glu Leu Pro Thr Lys Lys Gly Arg Arg Asn Arg Phe Lys Trp Gly Pro
        195                 200                 205

gca tcc cag cag atc ctg ttc cag gcc tat gag agg cag aag aac cct 672
Ala Ser Gln Gln Ile Leu Phe Gln Ala Tyr Glu Arg Gln Lys Asn Pro
    210                 215                 220

agc aag gag gag cga gag acg cta gtg gag gag tgc aat agg gcg gaa 720
Ser Lys Glu Glu Arg Glu Thr Leu Val Glu Glu Cys Asn Arg Ala Glu
225                 230                 235                 240

tgc atc cag aga ggg gtg tcc cca tca cag gca cag ggg ctg ggc tcc 768
Cys Ile Gln Arg Gly Val Ser Pro Ser Gln Ala Gln Gly Leu Gly Ser
                245                 250                 255

aac ctc gtc acg gag gtg cgt gtc tac aac tgg ttt gcc aac cgg cgc 816
Asn Leu Val Thr Glu Val Arg Val Tyr Asn Trp Phe Ala Asn Arg Arg
            260                 265                 270

aaa gaa gaa gcc ttc cgg cac aag ctg gcc atg gac acg tac agc ggg 864
Lys Glu Glu Ala Phe Arg His Lys Leu Ala Met Asp Thr Tyr Ser Gly
        275                 280                 285

ccc ccc cca ggg cca ggc ccg gga cct gcg ctg ccc gct cac agc tcc 912
Pro Pro Pro Gly Pro Gly Pro Gly Pro Ala Leu Pro Ala His Ser Ser
    290                 295                 300

cct ggc ctg cct cca cct gcc ctc tcc ccc agt aag gtc cac ggt gtg 960
Pro Gly Leu Pro Pro Pro Ala Leu Ser Pro Ser Lys Val His Gly Val
305                 310                 315                 320

cgc tat gga cag cct gcg acc agt gag act gca gaa gta ccc tca agc 1008
Arg Tyr Gly Gln Pro Ala Thr Ser Glu Thr Ala Glu Val Pro Ser Ser
                325                 330                 335

agc ggc ggt ccc tta gtg aca gtg tct aca ccc ctc cac caa gtg tcc 1056
Ser Gly Gly Pro Leu Val Thr Val Ser Thr Pro Leu His Gln Val Ser
            340                 345                 350

ccc acg ggc ctg gag ccc agc cac agc ctg ctg agt aca gaa gcc aag 1104
Pro Thr Gly Leu Glu Pro Ser His Ser Leu Leu Ser Thr Glu Ala Lys
        355                 360                 365

ctg gtc tca gca gct ggg ggc ccc ctc ccc cct gtc agc acc ctg aca 1152
Leu Val Ser Ala Ala Gly Gly Pro Leu Pro Pro Val Ser Thr Leu Thr
    370                 375                 380

gca ctg cac agc ttg gag cag aca tcc cca ggc ctc aac cag cag ccc 1200
Ala Leu His Ser Leu Glu Gln Thr Ser Pro Gly Leu Asn Gln Gln Pro
385                 390                 395                 400

cag aac ctc atc atg gcc tca ctt cct ggg gtc atg acc atc ggg cct 1248
Gln Asn Leu Ile Met Ala Ser Leu Pro Gly Val Met Thr Ile Gly Pro
                405                 410                 415

ggt gag cct gcc tcc ctg ggt cct acg ttc acc aac aca ggt gcc tcc 1296
Gly Glu Pro Ala Ser Leu Gly Pro Thr Phe Thr Asn Thr Gly Ala Ser
            420                 425                 430

acc ctg gtc atc ggc ctg gcc tcc acg cag gca cag agt gtg ccg gtc 1344
Thr Leu Val Ile Gly Leu Ala Ser Thr Gln Ala Gln Ser Val Pro Val
        435                 440                 445

atc aac agc atg ggc agc agc ctg acc acc ctg cag ccc gtc cag ttc 1392
Ile Asn Ser Met Gly Ser Ser Leu Thr Thr Leu Gln Pro Val Gln Phe
    450                 455                 460

tcc cag ccg ctg cac ccc tcc tac cag cag ccg ctc atg cca cct gtg 1440
Ser Gln Pro Leu His Pro Ser Tyr Gln Gln Pro Leu Met Pro Pro Val
465                 470                 475                 480

cag agc cat gtg acc cag aac ccc ttc atg gcc acc atg gct cag ctg 1488
Gln Ser His Val Thr Gln Asn Pro Phe Met Ala Thr Met Ala Gln Leu
                485                 490                 495

cag agc ccc cac gcc ctc tac agc cac aag ccc gag gtg gcc cag tac 1536
Gln Ser Pro His Ala Leu Tyr Ser His Lys Pro Glu Val Ala Gln Tyr
            500                 505                 510

acc cac acg ggc ctg ctc ccg cag act atg ctc atc acc gac acc acc 1584
Thr His Thr Gly Leu Leu Pro Gln Thr Met Leu Ile Thr Asp Thr Thr
        515                 520                 525

aac ctg agc gcc ctg gcc agc ctc acg ccc acc aag cag gtc ttc acc 1632
Asn Leu Ser Ala Leu Ala Ser Leu Thr Pro Thr Lys Gln Val Phe Thr
    530                 535                 540

tca gac act gag gcc tcc agt gag tcc ggg ctt cac acg ccg gca tct 1680
Ser Asp Thr Glu Ala Ser Ser Glu Ser Gly Leu His Thr Pro Ala Ser
545                 550                 555                 560

cag gcc acc acc ctc cac atc ccc agc cag gac cct gcc ggc atc cag 1728
Gln Ala Thr Thr Leu His Ile Pro Ser Gln Asp Pro Ala Gly Ile Gln
                565                 570                 575

cac ctg cag ccg gcc cac cgg ctc agc gcc agc ccc aca gtg tcc tcc 1776
His Leu Gln Pro Ala His Arg Leu Ser Ala Ser Pro Thr Val Ser Ser
            580                 585                 590

agc agc ctg gtg ctg tac cag agc tca gac tcc agc aat ggc cag agc 1824
Ser Ser Leu Val Leu Tyr Gln Ser Ser Asp Ser Ser Asn Gly Gln Ser
        595                 600                 605

cac ctg ctg cca tcc aac cac agc gtc atc gag acc ttc atc tcc acc 1872
His Leu Leu Pro Ser Asn His Ser Val Ile Glu Thr Phe Ile Ser Thr
    610                 615                 620

cag atg gcc tct tcc tcc cag taa 1896
Gln Met Ala Ser Ser Ser Gln
625                 630

<210> 2
<211> 631
<212> PRT
<213> Homo sapiens

<400> 2
Met Val Ser Lys Leu Ser Gln Leu Gln Thr Glu Leu Leu Ala Ala Leu
1               5                   10                  15

Leu Glu Ser Gly Leu Ser Lys Glu Ala Leu Ile Gln Ala Leu Gly Glu
            20                  25                  30

Pro Gly Pro Tyr Leu Leu Ala Gly Glu Gly Pro Leu Asp Lys Gly Glu
        35                  40                  45

Ser Cys Gly Gly Gly Arg Gly Glu Leu Ala Glu Leu Pro Asn Gly Leu
    50                  55                  60

Gly Glu Thr Arg Gly Ser Glu Asp Glu Thr Asp Asp Asp Gly Glu Asp
65                  70                  75                  80

Phe Thr Pro Pro Ile Leu Lys Glu Leu Glu Asn Leu Ser Pro Glu Glu
                85                  90                  95

Ala Ala His Gln Lys Ala Val Val Glu Thr Leu Leu Gln Glu Asp Pro
            100                 105                 110

Trp Arg Val Ala Lys Met Val Lys Ser Tyr Leu Gln Gln His Asn Ile
        115                 120                 125

Pro Gln Arg Glu Val Val Asp Thr Thr Gly Leu Asn Gln Ser His Leu
    130                 135                 140

Ser Gln His Leu Asn Lys Gly Thr Pro Met Lys Thr Gln Lys Arg Ala
145                 150                 155                 160

Ala Leu Tyr Thr Trp Tyr Val Arg Lys Gln Arg Glu Val Ala Gln Gln
                165                 170                 175

Phe Thr His Ala Gly Gln Gly Gly Leu Ile Glu Glu Pro Thr Gly Asp
            180                 185                 190

Glu Leu Pro Thr Lys Lys Gly Arg Arg Asn Arg Phe Lys Trp Gly Pro
        195                 200                 205

Ala Ser Gln Gln Ile Leu Phe Gln Ala Tyr Glu Arg Gln Lys Asn Pro
    210                 215                 220

Ser Lys Glu Glu Arg Glu Thr Leu Val Glu Glu Cys Asn Arg Ala Glu
225                 230                 235                 240

Cys Ile Gln Arg Gly Val Ser Pro Ser Gln Ala Gln Gly Leu Gly Ser
                245                 250                 255

Asn Leu Val Thr Glu Val Arg Val Tyr Asn Trp Phe Ala Asn Arg Arg
            260                 265                 270

Lys Glu Glu Ala Phe Arg His Lys Leu Ala Met Asp Thr Tyr Ser Gly
        275                 280                 285

Pro Pro Pro Gly Pro Gly Pro Gly Pro Ala Leu Pro Ala His Ser Ser
    290                 295                 300

Pro Gly Leu Pro Pro Pro Ala Leu Ser Pro Ser Lys Val His Gly Val
305                 310                 315                 320

Arg Tyr Gly Gln Pro Ala Thr Ser Glu Thr Ala Glu Val Pro Ser Ser
                325                 330                 335

Ser Gly Gly Pro Leu Val Thr Val Ser Thr Pro Leu His Gln Val Ser
        340                 345                 350

Pro Thr Gly Leu Glu Pro Ser His Ser Leu Leu Ser Thr Glu Ala Lys
    355                 360                 365

Leu Val Ser Ala Ala Gly Gly Pro Leu Pro Pro Val Ser Thr Leu Thr
    370                 375                 380

Ala Leu His Ser Leu Glu Gln Thr Ser Pro Gly Leu Asn Gln Gln Pro
385                 390                 395                 400

Gln Asn Leu Ile Met Ala Ser Leu Pro Gly Val Met Thr Ile Gly Pro
                405                 410                 415

Gly Glu Pro Ala Ser Leu Gly Pro Thr Phe Thr Asn Thr Gly Ala Ser
            420                 425                 430

Thr Leu Val Ile Gly Leu Ala Ser Thr Gln Ala Gln Ser Val Pro Val
        435                 440                 445

Ile Asn Ser Met Gly Ser Ser Leu Thr Thr Leu Gln Pro Val Gln Phe
    450                 455                 460

Ser Gln Pro Leu His Pro Ser Tyr Gln Gln Pro Leu Met Pro Pro Val
465                 470                 475                 480

Gln Ser His Val Thr Gln Asn Pro Phe Met Ala Thr Met Ala Gln Leu
                485                 490                 495

Gln Ser Pro His Ala Leu Tyr Ser His Lys Pro Glu Val Ala Gln Tyr
            500                 505                 510

Thr His Thr Gly Leu Leu Pro Gln Thr Met Leu Ile Thr Asp Thr Thr
        515                 520                 525

Asn Leu Ser Ala Leu Ala Ser Leu Thr Pro Thr Lys Gln Val Phe Thr
    530                 535                 540

Ser Asp Thr Glu Ala Ser Ser Glu Ser Gly Leu His Thr Pro Ala Ser
545                 550                 555                 560

Gln Ala Thr Thr Leu His Ile Pro Ser Gln Asp Pro Ala Gly Ile Gln
                565                 570                 575

His Leu Gln Pro Ala His Arg Leu Ser Ala Ser Pro Thr Val Ser Ser
            580                 585                 590

Ser Ser Leu Val Leu Tyr Gln Ser Ser Asp Ser Ser Asn Gly Gln Ser
        595                 600                 605

His Leu Leu Pro Ser Asn His Ser Val Ile Glu Thr Phe Ile Ser Thr
    610                 615                 620

Gln Met Ala Ser Ser Ser Gln
625                 630

<210>   3
<211>   93
<212>   DNA
<213>   Homo sapiens

<400>    3
gtaaggtcca ggcctgctgg ccctcccttg gcctgtgaca gagcccctca cccccacatc    60

ccccgggctc aggaggctgc tctgctcccc cag                                 93


<210>    4
<211>    41
<212>    DNA
<213>    Artificial Sequence

<220>
<223>    sense primer for amplifying promoter of MODY3 gene


<400>    4
taatacgact cactataggg tggccgtgag catcctctgc c                        41


<210>    5
<211>    39
<212>    DNA
<213>    Artificial Sequence

<220>
<223>    antisense primer for amplifying promoter of MODY3 gene


<400>    5
gtaaccctca ctaaagggac gtgggttgcg tttgcctgc                           39


<210>    6
<211>    40
<212>    DNA
<213>    Artificial Sequence

<220>
<223>    sense primer for amplifying exon 1 of MODY3 gene


<400>    6
taatacgact cactataggg cgtggccctg tggcagccga                          40


<210>    7
<211>    40
<212>    DNA
<213>    Artificial Sequence

<220>
<223>    antisense primer for amplifying exon 1 of MODY3 gene


<400>    7
gtaaccctca ctaaagggag ggctcgttag gagctgaggg                          40


<210>    8
<211>    42
<212>    DNA
<213>    Artificial Sequence

<220>
<223>    sense primer for amplifying exon 2 of MODY3 gene.


<400>    8
taatacgact cactataggg cccttgctga gcagatcccg tc                    42


<210>    9
<211>    40
<212>    DNA
<213>    Artificial Sequence

<220>
<223>    antisense primer for amplifying exon 2 of MODY3 gene


<400>    9
gtaaccctca ctaaagggag ggatggtgaa gcttccagcc                    40


<210>    10
<211>    40
<212>    DNA
<213>    Artificial Sequence

<220>
<223>    sense primer for amplifying exon 3 of MODY3 gene


<400>    10
taatacgact cactataggg gcaaggtcag gggaatggac                    40


<210>    11
<211>    42
<212>    DNA
<213>    Artificial Sequence

<220>
<223>    antisense primer for amplifying exon 3 of MODY3 gene


<400>    11
gtaaccctca ctaaagggac gccgttgtac ctattgcact cc                    42


<210>    12
<211>    43
<212>    DNA
<213>    Artificial Sequence

<220>
<223>    sense primer for amplifying exon 4 of MODY3 gene


<400>    12
taatacgact cactataggg ggctcatggg tggctatttc tgc                    43


<210>    13
<211>    42

<212>    DNA
<213>    Artificial Sequence

<220>
<223>    antisense primer for amplifying exon 4 of MODY3 gene


<400>    13
gtaaccctca ctaaagggac gtgtcccttg tccccacata cc                    42


<210>    14
<211>    42
<212>    DNA
<213>    Artificial Sequence

<220>
<223>    sense primer for amplifying exon 5 of MODY3 gene


<400>    14
taatacgact cactataggg tgctgaggca ggacactgct tc                    42


<210>    15
<211>    42
<212>    DNA
<213>    Artificial Sequence

<220>
<223>    antisense primer for amplifying exon 5 of MODY3 gene


<400>    15
gtaaccctca ctaaagggat acaagcaagg acactcacca gc                    42


<210>    16
<211>    41
<212>    DNA
<213>    Artificial Sequence

<220>
<223>    sense primer for amplifying exon 6 of MODY3 gene


<400>    16
taatacgact cactataggg cccggacaca gcttggcttc c                     41


<210>    17
<211>    42
<212>    DNA
<213>    Artificial Sequence

<220>
<223>    antisense primer for amplifying exon 6 of MODY3 gene


<400>    17
gtaaccctca ctaaagggaa tccccaccag cttaccgatg ac                    42

<210> 18
<211> 40
<212> DNA
<213> Artificial Sequence

<220>
<223> sense primer for amplifying exon 7 of MODY3 gene

<400> 18
taatacgact cactataggg caggcctggc ctccacgcag 40

<210> 19
<211> 40
<212> DNA
<213> Artificial Sequence

<220>
<223> antisense primer for amplifying exon 7 of MODY3 gene

<400> 19
gtaaccctca ctaaagggag gggctctgca gctgagccat 40

<210> 20
<211> 41
<212> DNA
<213> Artificial Sequence

<220>
<223> sense primer for amplifying exon 8 and 9 of MODY3 gene

<400> 20
taatacgact cactataggg ggcccagtac acccacacgg g 41

<210> 21
<211> 40
<212> DNA
<213> Artificial Sequence

<220>
<223> antisense primer for amplifying exon 8 and 9 of MODY3 gene

<400> 21
gtaaccctca ctaaagggag ggcagggaca gtaagggagg 40

<210> 22
<211> 41
<212> DNA
<213> Artificial Sequence

<220>
<223> sense primer for amplifying exon 10 of MODY3 gene

<400> 22
taatacgact cactataggg gccttgtttg cctctgcagt g 41

<210> 23
<211> 41
<212> DNA
<213> Artificial Sequence

<220>
<223> antisense primer for amplifying exon 10 of MODY3 gene

<400> 23
gtaaccctca ctaaagggag gccatctggg tggagatgaa g 41

<210> 24
<211> 20
<212> DNA
<213> Artificial Sequence

<220>
<223> T7 promoter sequence

<400> 24
taatacgact cactataggg 20

<210> 25
<211> 19
<212> DNA
<213> Artificial Sequence

<220>
<223> T3 promoter sequence

<400> 25
gtaaccctca ctaaaggga 19